# EUROPEAN PATENT SPECIFICATION

(11) **EP 2 488 198 B1**
(45) Date of publication and mention of the grant of the patent: **28.09.2016**
(21) Application number: 10823995.5
(22) Date of filing: 13.10.2010
(51) Int. Cl.: A61K 39/02, G01N 33/48, G01N 33/68, G01N 33/50, G01N 33/569, G01N 33/564, A61K 38/16

(54) **TREATMENT AND DIAGNOSIS OF AUTOIMMUNE DISORDERS**
BEHANDLUNG UND DIAGNOSE VON AUTOIMMUNERKRANKUNGEN
TRAITEMENT ET DIAGNOSTIC DE MALADIES AUTOIMMUNES

(30) Priority: 10.12.2009 US 285378 P; 13.10.2009 US 251171 P
(43) Date of publication of application: 22.08.2012
(73) Proprietor: Rutgers, the State University of New Jersey, New Brunswick, NJ 08909 (US); Actinobac Biomed, Inc., Kendall Park, NJ 08824 (US)
(72) Inventor: KACHLANY, Scott, C., Bridgewater NJ 08807 (US); BELINKA, Benjamin, A., Kendall Park NJ 08824 (US)
(74) Representative: Zacco GmbH
(86) International application number: PCT/US2010/052453
(87) International publication number: WO 2011/047011

(56) References cited:
- US-A1- 2005 032 217
- US-A1- 2005 032 217
- GUTTMAN-YASSKY EMMA ET AL: "Blockade of CD11a by efalizumab in psoriasis patients induces a unique state of T-cell hyporesponsiveness.", THE JOURNAL OF INVESTIGATIVE DERMATOLOGY MAY 2008, vol. 128, no. 5, May 2008 (2008-05), pages 1182-1191, XP002712650, ISSN: 1523-1747
- KACHLANY S C ET AL: "Anti-leukemia activity of a bacterial toxin with natural specificity for LFA-1 on white blood cells", LEUKEMIA RESEARCH, NEW YORK,NY, US, vol. 34, no. 6, 10 September 2009 (2009-09-10), pages 777-785, XP027015269, ISSN: 0145-2126 [retrieved on 2009-09-10]
- P. GIBLIN ET AL: "LFA-1 as a Key Regulator of Immune Function: Approaches toward the Development of LFA-1-Based Therapeutics", CURRENT PHARMACEUTICAL DESIGN, vol. 12, no. 22, 1 August 2006 (2006-08-01), pages 2771-2795, XP055078731, ISSN: 1381-6128, DOI: 10.2174/138161206777947731
- LUCY DESJARDIN ET AL: "Mycobacterium tuberculosis-infected human macrophages exhibit enhanced cellular adhesion with increased expression of LFA-1 and ICAM-1 and reduced expression and/or function of complement receptors, FcgammaRII and the mannose receptor.", MICROBIOLOGY, vol. 148, no. 10, 1 October 2002 (2002-10-01), pages 3161-3171, XP055078732, ISSN: 1350-0872
- SHAMIK GHOSH ET AL: "The LFA-1 adhesion molecule is required for protective immunity during pulmonary Mycobacterium tuberculosis infection.", THE JOURNAL OF IMMUNOLOGY, vol. 176, no. 8, 1 April 2006 (2006-04-01) , pages 4914-4922, XP055078733, ISSN: 0022-1767

## Description

### FIELD OF THE INVENTION

This invention relates to reagents and methods for treating or diagnosing autoimmune disorders.

### BACKGROUND OF THE INVENTION

Inflammatory disorders are disorders characterized by the abnormal activation and subsequent migration of white blood cells (WBCs) to affected areas of the body. These conditions encompass a wide range of ailments that affect the lives of millions of people throughout the world. Although some treatments are presently available, many possess significantly side effects or are not very effective in alleviating all symptoms. At the same time, few tests exist that reliably diagnose or monitor the progress of the diseases. Thus, there is a need for drugs and reagents for treatment and diagnosis of inflammatory disorders.

US2005/032217 discloses compositions (e.g. for topical application) and treatment of inflammatory and autoimmune disorders by leukotoxins, such as LtxA.

### SUMMARY OF THE INVENTION

This invention relates to treatment and diagnosis of autoimmune diseases, using leukotoxin (LtxA), a bacterial protein. Shown below are the polypeptide and nucleotide sequences of LtxA.
*Aggregatibacter actinomycetemcomitans* strain NJ4500 protein sequence (SEQ ID NO: 1) 
MATTSLLNTKQQAAQFANSVADRAKENIDAAKEQLQKALDKLGKTGKKLTLYIKNYKKGN
GLTALIKAAQKLGIEVYHEGKDGPALTNGILNTGKKLLGLTERGLTLFAPELDKWIQGNK
HLSNSVGSTGNLTKAIDKVQSVLGTLQAFLNTAFSGMDLDALIKARQNGKNVTDVQLAKA
SLNLINELIGTISSITNNVDTFSKQLNKLGEALGQVKHFGSFGDKLKNLPKLGNLGKGLG
ALSGVLSAISAALLLANKDADTATKAAAAAELTNKVLGNIGKAITQYLIAQRAAAGLSTT
GPVAGLIASVVSLAISPLSFLGIAKQFDRARMLEEYSKRFKKFGYNGDSLLGQFYKNTGI
ADAAITTINTVLSAIAAGVGAASAGSLVGAPIGLLVSAITSLISGILDASKQAVFEHIAN
QLADKIKAWENKYGKNYFENGYDARHSAFLEDSLKLFNELREKYKTENILSITQQGWDQR
IGELAGITRNGDRIQSGKAYVDYLKKGEELAKHSDKFTKQILDPIKGNIDLSGIKGSTTL
TFLNPLLTAGKEERKTRQSGKYEFITELKVKGRTDWKVKGVPNSNGVYDFSNLIQHAVTR
DNKVLEARLIANLGAKDDYVFVGSGSTIVNAGDGYDVVDYSKGRTGALTIDGRNATKAGQ
YKVERDLSGTQVLQETVSKQETKRGKVTDLLEYRNYKLDYYYTNKGFKAHDELNSVEEII
GSTLRDKFYGSKFNDVFHGHDGDDLIYGYDGDDRLYGDNGNDEIHGGQGNDKLYGGAGND
RLFGEYGNNYLDGGEGDDHLEGGNGSDILRGGSGNDKLFGNQGDDLLDGGEGDDQLAGGE
GNDIYVYRKEYGHHTITEHSGDKDKLSLANINLKDVSFERNGNDLLLKTNNRTAVTFKGW
FSKPNSSAGLDEYQRKLLEYAPEKDRARLKRQFELQRGKVDKSLNNKVEEIIGKDGERIT
SQDIDNLFDKSGNKKTISPQELAGLIKNKGKSSSLMSSSRSSSMLTQKSGLSNDISRIIS
ATSGFGSSGKALSASPLQTNNNFNSYANSLATTAA
*Aggregatibacter actinomycetemcomitans* strain NJ4500 DNA sequence (SEQ ID NO: 2)

ATGGCAACTACTTCACTGCTAAATACAAAACAGCAAGCTGCACAGTTTGCAAATTCAGTT
GCAGATAGAGCTAAGGAAAATATTGATGCTGCAAAAGAACAATTGCAAAAGGCGTTAGAT
AAATTAGGGAAGACAGGTAAGAAATTAACTTTATATATCCCTAAGAATTACAAAAAAGGA
AATGGTCTTACTGCGCTTATAAAAGCAGCACAGAAGTTAGGGATTGAAGTATATCATGAA
GGGAAAGACGGCCCGGCATTAACTAATGGTATTTTAAATACTGGGAAAAAATTACTTGGT
CTTACCGAACGAGGTTTAACTTTATTTGCTCCGGAATTAGATAAATGGATTCAAGGTAAT
AAACATTTAAGTAATTCTGTGGGTAGTACTGGAAATTTGACAAAAGCGATAGATAAGGTT
CAGAGTGTTCTTGGTACGTTACAAGCGTTTTTGAACACCGCATTTTCGGGCATGGATTTA
GATGCCTTAATTAAAGCCCGTCAAAATGGTAAAAATGTAACAGATGTACAGCTAGCAAAA
GCCAGTCTTAACCTGATTAATGAATTGATTGGTACTATTTCTAGCATTACAAATAATGTA
GATACTTTTTCTAAACAACTTAATAAGTTAGGTGAAGCACTAGGACAAGTAAAACATTTT
GGTAGTTTTGGAGATAAATTAAAGAATTTACCTAAGTTAGGTAATCTTGGAAAAGGTTTA
GGTGCATTATCCGGTGTATTGTCGGCTATATCAGCGGCTCTATTACTTGCAAATAAAGAT
GCTGATACTGCAACGAAAGCAGCGGCTGCAGCTGAATTGACAAATAAAGTGCTAGGTAAC
ATCGGTAAAGCGATCACACAATACTTGATTGCTCAACGTGCTGCAGCGGGGcTTTCTACT
ACGGGACCTGTCGCAGGGTTAATTGCCTCTGTGGTCAGCTTGGCAATCAGCCCTTTGTCT
TTCCTAGGTATTGCGAAACAATTTGATCGTGCGAGAATGCTTGAGGAATACTCGAAACGC
TTTAAGAAATTTGGTTATAACGGCGATAGTTTACTTGGTCAATTCTACAAAAATACAGGG
ATCGCAGATGCTGCGATTACAACGATTAACACTGTATTAAGTGCTATTGCAGCAGGGGTT
GGTGCAGCCTCCGCCGGTTCTTTAGTTGGTGCGCCAATCGGTTTGTTAGTGAGTGCGATT
ACCAGCTTAATTTCAGGAATTCTTGATGCTTCTAAACAAGCCGTTTTTGAACATATCGCG
AATCAGCTCGCCGATAAAATTAAAGCATGGGAGAATAAGTACGGTAAGAATTACTTTGAA
AATGGCTATGATGCCCGTCATTCCGCCTTCTTGGAAGATTCACTAAAATTATTTAATGAG
TTACGTGAAAAATATAAAACCGAAAATATATTATCTATCACTCAACAAGGTTGGGATCAG
CGCATTGGTGAATTAGCAGGTATCACTCGTAATGGAGATCGTATTCAAAGTGGTAAAGCT
TATGTGGATTATTTGAAAAAGGGTGAGGAGCTTGCAAAGCATAGCGATAAATTCACTAAA
CAGATTTTAGATCCAATCAAAGGTAATATTGATCTTTCGGGTATaAAAGGTTCTACCACT

CTAACTTTTTAAATCCGTTGTTAACCGCAGGTAAGGAAGAACGGAAAACACGTCAGTCA
GGTAAATATGAATTTATTACTGAATTAAAAGTAAAAGGACGTACCGATTGGAAGGTAAAA
GGTGTTCCTAATTCTAATGGTGTATATGATTTTTCTAACTTAATTCAACATGCCGTTACA
CGTGATAATAAAGTTCTAGAAGCAAGATTAATTGCTAATTTGGGTGCTAAAGATGATTAT
GTTTTTGTCGGATCCGGTTCAACAATAGTTAATGCTGGAGACGGTTATGATGTGGTGGAC
TATAGTAAAGGTCgCACCGGTGCATTAACAATCGACGGTCGTAATGCTACTAAAGCCGGA
CAATATAAGGTTGAAAGAGATCTTAGCGGTACTCAAGTCTTGCAGGAAACCGTATCAAAG
CAAGAAACTAAACGAGGGAAGGTTACCGATCTACTTGAATATCGTAACTATAAATTAGAT
TACTATTATACGAATAAGGGCTTTAAAGCTCATGATGAATTAAACTCAGTAGAGGAAATT
ATCGGCAGCACACTACGTGATAAATTTTATGGTTCTAAATTTAATGATGTTTTCCATGGT
CACGATGGCGATGATTTGATTTATGGTTATGATGGCGATGATCGTTTGTATGGCGATAAT
GGGAATGACGAAATTCATGGCGGCCAAGGTAATGATAAGCTCTATGGTGGTGCCGGTAAC
GATAGGCTCTTTGGTGAATATGGCAACAACTATCTTGACGGTGGAGAAGGCGACGACCAC
TTAGAGGGAGGCAATGGTTCCGATATTCTAAGAGGTGGAAGTGGCAATGATAAGTTGTTT
GGAAACCAAGGAGATGATTTACTTGACGGTGGAGAAGGCGATGACCAACTTGCCGGTGGA
GAAGGAAATGATATTTATGTTTACCGTAAAGAATATGGGCACCACACTATTACGGAACAT
AGCGGTGATAAAGATAAATTATCATTAGCAAATATCAATCTCAAAGATGTGTCATTTGAG
CGTAACGGCAATGATCTACTATTGAAAACAAATAATAGAACAGCAGTAACATTTAAAGGA
TGGTTTAGTAAACCTAATTCATCGGCAGGATTAGATGAGTATCAAAGAAAACTTCTTGAA
TACGCACCTGAAAAGGATCGTGCACGACTTAAGAGACAATTTGAGTTACAGCGAGGTAAA
GTCGACAAATCACTCAATAATAAAGTTGAAGAAATTATCGGTAAAGATGGGGAGCGGATT
ACTTCGCAAGACATTGATAATCTTTTTGATAAGAGTGGGAACAAAAAGACAATTTCACCT
CAAGAGCTTGCCGGACTTATTAAGAATAAAGGTAAGTCAAGTAGCCTTATGTCTTCTTCT
CGTTCGTCAAGTATGCTTACACAAAAGTCCGGTTTGTCAAATGATATTAGTCGTATTATT
TCAGCAACCAGTGGTTTTGGTTCATCCGGTAAAGCGTTATCCGCTTCGCCATTGCAGACC
AATAATAACTTTAACTCTTACGCAAATTCGTTAGCAACTACTGCGGCC

The invention is defined by the claims.

### DESCRIPTION OF DRAWINGS

FIG. 1 is a set of diagrams showing sensitivity of Jurkat-derived cells (A) and PBMCs (B) to LtxA-mediated cytotoxicity.
FIG. 2 is a set of diagrams showing flow cytometry results of healthy human PBMCs' staining with anti-LFA-1 antibody and sensitivity to LtxA.
FIG. 3 is a set of diagrams showing flow cytometry results of cells staining with LtxA-FITC.
FIG. 4 is a set of diagrams showing binding of activated PBMCs to brain endothelial cells (HBECs).
FIG. 5 is a diagram showing migration of WBCs across a brain endothelial cell barrier.
FIG. 6 is a set of diagrams showing effects of LtxA and Efalizumab on proliferation of T-cells.
FIG. 7 is a set of diagrams showing effects of LtxA and Efalizumab on proliferation of PBMCs from psoriasis patients.
FIG. 8 is a set of diagrams showing effects of LtxA and Efalizumab on semiquantitative clinical psoriasis score and epidermal thickness in psoriasis xenograft transplantation models.
FIG. 9 is a set of diagrams showing effects of LtxA and Efalizumab on psoriasis pattern scores and parakeratosis scores in the psoriasis xenograft transplantation models.
FIG. 10 is a set of diagrams showing effects of LtxA and Efalizumab on angiogenesis scores and lymphocyte scores in the psoriasis xenograft transplantation models.
FIG. 11 is a diagram showing effects of LtxA and Efalizumab on stratum granulosum scores in the psoriasis xenograft transplantation models.

### DETAILED DESCRIPTION OF THE INVENTION

This invention relates to reagents and methods for treating or diagnosing autoimmune diseases. Such diseases are characterized by the chronic activation of immune cells under non-activating conditions. A major player in the etiology of these conditions is leukocyte function antigen-1 (LFA-1).

LFA-1, a β2-integrin on the surface of white blood cells, is composed of CD11a and CD18 and involved in immune cell migration and signaling. In the absence of infection, circulating WBCs express a "resting state" LFA-1 on their surface. These WBCs play an essential role in immune surveillance, waiting to be called upon by the immune system. During an infection, WBCs need to migrate to the site of insult to destroy the invading pathogens. Extravasation of WBCs into the infected tissue is mediated by signals, such as cytokines, that are released by host cells at the infection site. Inflammatory cytokines cause LFA-1 to assume an active conformation, which results in binding of activated LFA-1 to intercellular adhesion molecule-1 (ICAM-1) on the surface of endothelial cells. The interaction between LFA-1 and ICAM-1 results in migration of WBCs across the endothelial barrier and into the infected tissue. As LFA-1 is involved in the migration of immune cells to various sites of infection, the chronic activation and upregulation of LFA-1 results in the migration of these cells to various tissues of the body, resulting in inflammation and organ damage.

One of the most prevalent autoimmune diseases is psoriasis. This disease, for which there is no cure, affects 2-3% of the population (7-8 million in U.S.; 125 million worldwide). It results from the hyper-activation of immune cells and keratinocytes in the dermis. The immune cells involved in psoriatic lesions have an upregulation and activation of LFA-1. Efalizumab (RAPTIVA), a recombinant monoclonal antibody which binds to the CD11 subunit of LFA-1, was approved for clinical use in patients with this condition but has been withdrawn from the market due to increased risk of viral infection of the central nervous system (progressive multifocal leukoencephalopathy), bacteria sepsis, invasive fungal disease, and other opportunistic infections.

GUTTMAN-YASSKY EMMA ET AL: "Blockade of CD11a by Efalizumab in psoriasis patients induces a unique state of T-cell hyporesponsiveness.", THE JOURNAL OF INVESTIGATIVE DERMATOLOGY MAY 2008, vol. 128, no. 5, pages 1182-1191, teaches that Efalizumab (anti-CD11a) interferes with LFA-1/ICAM-1 binding and inhibits several key steps in psoriasis pathogenesis. P. GIBLIN ET AL: "LFA-1 as a Key Regulator of Immune Function: Approaches toward the Development of LFA-1-Based Therapeutics", CURRENT PHARMACEUTICAL DESIGN, vol. 12, no. 22, 1 August 2006, pages 2771-2795, describes LFA-1 as a key regulator of immune function and approaches toward the development of LFA-1-based therapeutics, e.g. for psoriasis.

Current treatment for psoriasis includes topical therapy, phototherapy, and systemic administration of steroids and biologics. Many of the therapeutic options are not highly targeted (e.g., steroids, anthralin) however, and exhibit toxic side effects (e.g., biologics, cyclosporine, methotrexate, retinoids). Indeed, the most effective therapies are also considered to have the greatest adverse reactions. Furthermore, over time, psoriasis can become resistant to specific therapies and these treatments are periodically changed to prevent both the development of drug resistance and the occurrence of adverse reactions. This practice is known as treatment rotation.

This invention is based, at least in part, on the fact that LtxA efficiently and specifically targets and kills WBCs that express the activated conformation of LFA-1 on their surface while having little or no toxic effect on other cells or organs in the body. As disclosed in the examples below, LtxA is highly effective in treating psoriasis with minimal toxicity because of its target specificity.

### LtxA

LtxA is a ∼115 kDa protein produced by the Gram negative bacterium *Aggregatibacter actinomycetemcomitans* (Kachlany, S. C. 2010. J Dent Res 89:561-570.). LtxA specifically kills leukocytes of humans and Old World Primates by forming pores in the membrane and causing apoptosis or necrosis (Mangan et al., 1991. Infect Immun 59:3267-72.). LtxA binds specifically to LFA-1 and cells that lack LFA-1 are resistant to its toxicity (Kachlany, S. C. et al., 2010. Leukemia Research 34:777-85.). For example, LtxA is not active against human red blood cells, human epithelial cells, rat cells, or mouse cells. LtxA also remains active in the presence of human peripheral blood.

Since LtxA is able to identify and kill white blood cells resulting from autoimmune disease, it is an ideal agent for both the detection and treatment of these conditions. For example, blood from a patient can be analyzed using LtxA-FITC staining. A finding of a large percentage of activated WBCs indicates that the patient should undergo LtxA therapy. The effectiveness of the leukotoxin treatments can be monitored by employing LtxA-FITC reagent that initially diagnosed the disease. As the patient responds positively to treatment, the number of WBCs with upregulated activated surface LFA-1 should be seen to decrease. Further, because of LtxA's highly specific targeting ability, few side effects are expected.

LtxA is able to kill many leukemia and lymphoma cell lines and preclinical studies have shown that it may be an effective targeted therapy for treating hematological malignancies. LtxA is expected to be more advantageous than Efalizumab because it displays a significantly greater selective action by only targeting active LFA-1 and thereby mainly destroying activated leukocytes involved in disease. In non-human primates, it was found that a single LtxA treatment depleted leukocyte counts for only 12 hours and high doses administered to mice were found to be non-toxic.

While many LtxA preparations can be used, highly purified LtxA is preferred. Examples include LtxA polypeptide purified from *Aggregatibacter actinomycetemcomitans* (SEQ ID NO: 1 shown above) and other variants having substantially the same biological activity as that having the sequence of SEQ ID NO: 1. It was discovered that *Aggregatibacter actinomycetemcomitans* secreted active LtxA into culture supernatants (Kachlany, S. C., et al. 2000. Infect Immun 68:6094-100) and an efficient method for its purification was described in Kachlany, S. C., et al. 2002. Protein Expr Purif 25:465-71. This method can therefore be used to prepare isolated or purified LtxA polypeptide. In one example, a purification procedure of the toxin involves:
a. inoculating a single colony of *Aggregatibacter actinomycetemcomitans* into a fresh broth and growing cultures;
b. adding the growing cultures to fresh broth, adding glass beads and incubating;
c. centrifuging the incubated culture, forming a pellet and a supernatant;
d. filtering the supernatant through a membrane to provided a filtered supernatant;
e. mixing (NH₄)₂SO₄ and the filtered supernatant together to form a mixture;
f. centrifuging the mixture to form a mixture pellet;
g. resuspending the mixture pellet in buffer to form a protein resuspension;
h. passing the protein resuspension through a column; and
i. collecting the protein eluting off the column.
See also PCT/US2006/45258 (WO 2007/062150) and US Application 20090075883 (US Ser. No . 12/154,843). The contents of these two documents are incorporated herein by reference.

An "isolated polypeptide" refers to a polypeptide that has been separated from other proteins, lipids, and nucleic acids with which it is naturally associated. The polypeptide can constitutes at least 10% (i.e., any percentage between 10% and 100%, e.g., 20%, 30%, 40%, 50%, 60%, 70 %, 80%, 85%, 90%, 95%, and 99%) by dry weight of the purified preparation. Purity can be measured by any appropriate standard method, for example, by column chromatography, polyacrylamide gel electrophoresis, or HPLC analysis. An isolated polypeptide of the invention can be purified from a natural source, produced by recombinant DNA techniques, or by chemical methods. A functional equivalent of LtxA refers to a polypeptide derivative of the LtxA polypeptide, e.g., a protein having one or more point mutations, insertions, deletions, truncations, a fusion protein, or a combination thereof. It retains substantially the activity of the LtxA polypeptide, i.e., the ability to target and kill WBCs that express the activated conformation of LFA-1 on their surface while having little or no toxic effect on other cells or organs in the body. The isolated polypeptide can contain SEQ ID NO: 1 or a functional fragment of SEQ ID NO: 1. In general, the functional equivalent is at least 95% identical to SEQ ID NO: 1.

All of naturally occurring LtxA, genetic engineered LtxA, and chemically synthesized LtxA can be used to practice the invention disclosed therein. LtxA obtained by recombinant DNA technology may have the same amino acid sequence as naturally a occurring LtxA (SEQ ID NO: 1) or an functionally equivalent thereof. The term "LtxA" also covers chemically modified LtxA. Examples of chemically modified LtxA include LtxA subjected to conformational change, addition or deletion of a sugar chain, and LtxA to which a compound such as polyethylene glycol has been bound. Once purified and tested by standard methods or according to the method described in the examples below, LtxA can be included in pharmaceutical composition, e.g., a topical composition.

The amino acid composition of the LtxA polypeptide described herein may vary without disrupting the ability of the polypeptide to target and kill WBCs. For example, it can contain one or more conservative amino acid substitutions. A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Thus, a predicted nonessential amino acid residue in SEQ ID NO: 1 is preferably replaced with another amino acid residue from the same side chain family. Alternatively, mutations can be introduced randomly along all or part of SEQ ID NO: 1, such as by saturation mutagenesis, and the resultant mutants can be screened for the ability to improve skin condition to identify mutants that retain the activity as described below in the examples.

A LtxA polypeptide as described in this invention can be obtained as a naturally occurring polypeptide or a recombinant polypeptide. To prepare a recombinant polypeptide, a nucleic acid encoding it (e.g., SEQ ID NO: 2) can be linked to another nucleic acid encoding a fusion partner, e.g., glutathione-s-transferase (GST), 6x-His epitope tag, or M13 Gene 3 protein. The resultant fusion nucleic acid expresses in suitable host cells a fusion protein that can be isolated by methods known in the art. The isolated fusion protein can be further treated, e.g., by enzymatic digestion, to remove the fusion partner and obtain the recombinant polypeptide of this invention.

### Compositions

Within the scope of this invention is a composition that contains a suitable carrier and one or more of the active agents described above, e.g., LtxA. The composition can be a pharmaceutical composition that contains a pharmaceutically acceptable carrier, or a cosmetic composition that contains a cosmetically acceptable carrier.

The term "pharmaceutical composition" refers to the combination of an active agent with a carrier, inert or active, making the composition especially suitable for diagnostic or therapeutic use in vivo or ex vivo. The term "pharmaceutically acceptable carrier" refers to any of the standard pharmaceutical carriers, such as a phosphate buffered saline solution, water, emulsions, and various types of wetting agents. The compositions also can include stabilizers and preservatives. A pharmaceutically acceptable carrier, after administered to or upon a subject, does not cause undesirable physiological effects. The carrier in the pharmaceutical composition must be "acceptable" also in the sense that it is compatible with the active ingredient and, preferably, capable of stabilizing it. One or more solubilizing agents can be utilized as pharmaceutical carriers for delivery of an active agent. Examples of other carriers include colloidal silicon oxide, magnesium stearate, cellulose, and sodium lauryl sulfate.

Pharmaceutical compositions for topical administration according to the present invention can be formulated as solutions, ointments, creams, suspensions, lotions, powders, pastes, gels, sprays, aerosols, or oils. Alternatively, topical formulations can be in the form of patches or dressings impregnated with active ingredient(s), which can optionally comprise one or more excipients or diluents. In some preferred embodiments, the topical formulations include a material that would enhance absorption or penetration of the active agent(s) through the skin or other affected areas.

A topical composition contains a safe and effective amount of a dermatologically acceptable carrier suitable for application to the skin. A "cosmetically acceptable" or "dermatologically-acceptable" composition or component refers a composition or component that is suitable for use in contact with human skin without undue toxicity, incompatibility, instability, allergic response, and the like. The carrier enables an active agent (such as LtxA) and optional component to be delivered to the skin at an appropriate concentration(s). The carrier can thus act as a diluent, dispersant, solvent, or the like to ensure that the active materials are applied to and distributed evenly over the selected target at an appropriate concentration. The carrier can be solid, semi-solid, or liquid. Preferably, it is in the form of a lotion, a cream, or a gel, in particular one that has a sufficient thickness or yield point to prevent the active materials from sedimenting. The carrier can be inert or possess dermatological benefits of its own. It should also be physically and chemically compatible with the active components described herein, and should not unduly impair stability, efficacy, or other use benefits associated with the composition.

The topical composition may be a cosmetic or dermatologic product in the form known in the art for topical or transdermal applications, including solutions, aerosols, creams, gels, patches, ointment, lotion, or foam.

The cosmetic composition may contain a wide variety of optional components, provided that such optional components are physically and chemically compatible with the essential components described herein. Examples of such components include those described in, e.g., U.S. Patent No. 7405195; Harry's Cosmeticology, 7th Ed., Harry & Wilkinson (Hill Publishers, London 1982); Pharmaceutical Dosage Forms--Disperse Systems; Lieberman, Rieger & Banker, Vols. 1 (1988) & 2 (1989); Marcel Decker,. Inc.; The Chemistry and Manufacture of Cosmetics, 2nd. Ed., deNavarre (Van Nostrand 1962-1965); and The Handbook of Cosmetic Science and Technology, 1st Ed. Knowlton & Pearce (Elsevier 1993).

The topical composition is useful for treating inflammatory disorders in the skin, such as psoriasis.

### Treatment Methods

The above-described active agents or a composition containing the agents can be used to treat an autoimmune disorder. Accordingly, the invention also features methods for treating in a subject an autoimmune disease. Autoimmune diseases are disorders characterized by the chronic activation of immune cells under non-activating conditions. Examples include psoriasis, inflammatory bowel diseases (e.g., Crohn's disease and ulcerative colitis), rheumatoid arthritis, multiple sclerosis, lupus and transplant rejection.

Among the above-listed diseases, psoriasis is the most prevalent autoimmune disease, affecting 2-3% of the population. Psoriasis is a disease that affects the dermis and results from the hyper-activation of immune cells and keratinocytes. It has been found that immune cells involved in psoriatic lesions have an upregulation of LFA-1 (de Boer et al., Archives of dermatological research 1994; 286: 304-311 and McGregor et al., Journal of the American Academy of Dermatology 1992: 27: 383-388.) In fact, Efalizumab (Raptiva) is a monoclonal antibody therapy that is indicated for the treatment of psoriasis. Thus, LtxA and Efalizumab both target the same cell type.

Crohn's disease and ulcerative colitis are diseases of the GI tract and result from an influx of immune cells into the intestines. Several studies have shown that LFA-1 is upregulated in colonic lymphocytes involved in the pathogenesis of Crohn's disease and ulcerative colitis (Bernstein et al., Clinical immunology, Orlando, Fla 2002; 104: 67-72 and Vainer et al., The American journal of surgical pathology 2000; 24: 1115-1124).

Multiple sclerosis (MS) is characterized by the influx of immune cells into the central nervous system. Studies have also shown upregulated expression of LFA-1 on immune cells collected from blood and cerebral spinal fluid from MS patients (Elovaara et al. Neurology 1998; 51: 1703-1708; and Elovaara et al., Archives of neurology 2000; 57: 546-551.). As described below in the example section, LtxA can suppress symptoms of MS via interfering with cell adhesion and deplete highly-activated immune cells in the CNS.

Rheumatoid arthritis is a disease affecting the joints and occurs when activated immune cells migrate to the synovium. Immune cells found in synovial fluid from RA patients have enhanced expression of LFA-1 and blocking LFA-1 has proven an effective therapeutic strategy in experimental animal systems (see, e.g., Singh et al., J Immunol 2008; 180: 1971-1978). Thus, LtxA would preferentially affect and eliminate the immune cells involved in RA pathogenesis.

Systemic lupus erythematosus or lupus is an autoimmune disease that affects primarily women and for which there is little effective treatment. The disease is characterized by immune complex-mediated tissue injury. Activated immune cells are responsible for the production of immune complexes. These immune cells express LFA-1 at high levels and several studies have shown the critical importance of LFA-1-expressing cells in the development of lupus (see, e.g., Kevil et al., The American journal of pathology; 2004; 165: 609-616.). Accordingly, LtxA can be used in treating systemic lupus erythematosus.

A "subject" refers to a human and a non-human animal. Examples of a non-human animal include all vertebrates, e.g., mammals, such as non-human primates (particularly higher primates), dog, rodent (e.g., mouse or rat), guinea pig, cat, and non-mammals, such as birds, amphibians, reptiles, etc. In a preferred embodiment, the subject is a human. In another embodiment, the subject is an experimental animal or animal suitable as a disease model.

A subject to be treated for an inflammatory disorder can be identified by standard diagnosing techniques for the disorder. Optionally, the subject can be examined for the level or percentage of WBCs that bind to LtxA in a test sample obtained from the subject by methods described below. If the binding level or percentage is at or above a threshold value (which can be obtained from a normal subject), the subject is a candidate for treatment with an effective amount of LtxA.

"Treating" or "treatment" refers to administration of a compound or agent to a subject, who has a disorder (such as an inflammatory disorder), with the purpose to cure, alleviate, relieve, remedy, delay the onset of, or ameliorate the disorder, the symptom of the disorder, the disease state secondary to the disorder, or the predisposition toward the disorder.
An "therapeutically effective amount" refers to the amount of an agent sufficient to effect beneficial or desired results. A therapeutically effective amount can be administered in one or more administrations, applications or dosages and is not intended to be limited to a particular formulation or administration route.

The agent can be administered *in vivo* or *ex vivo,* alone or co-administered in conjunction with other drugs or therapy. As used herein, the term "co-administration" or "co-administered" refers to the administration of at least two agent(s) or therapies to a subject. In some embodiments, the co-administration of two or more agents/therapies is concurrent. In other embodiments, a first agent/therapy is administered prior to a second agent/therapy. Those of skill in the art understand that the formulations and/or routes of administration of the various agents/therapies used may vary.

In an *in vivo* approach, LtxA is administered to a subject. Generally, LtxA is suspended in a pharmaceutically-acceptable carrier (e.g., physiological saline) and administered orally or by intravenous infusion, or injected or implanted subcutaneously, intramuscularly, intrathecally, intraperitoneally, intrarectally, intravaginally, intranasally, intragastrically, intratracheally, or intrapulmonarily.

The dosage required depends on the choice of the route of administration; the nature of the formulation; the nature of the patient's illness; the subject's size, weight, surface area, age, and sex; other drugs being administered; and the judgment of the attending physician. Suitable dosages are in the range of 0.01-100 mg/kg. Variations in the needed dosage are to be expected in view of the variety of compounds available and the different efficiencies of various routes of administration. Variations in these dosage levels can be adjusted using standard empirical routines for optimization as is well understood in the art. Encapsulation of the compound in a suitable delivery vehicle (e.g., polymeric microparticles or implantable devices) may increase the efficiency of delivery.

### Diagnostic and Prognostic Methods

As discussed above, LFA-1, present on WBCs of inflammatory disease patients, can act as a marker to detect and monitor the treatment of these afflictions while providing a therapeutic target for pharmaceutical agents. LtxA specifically targets WBCs that express the activated conformation of LFA-1, and therefore can be used in diagnosing diseases meditated by such WBCs.

To that end, this invention also features diagnosis methods. WBCs expressing the activated conformation of LFA-1 can be detected in a subject based on the presence of the binding of LtxA in a test sample from the subject. In other words, the binding of LtxA can be used as markers to indicate the presence or absence of WBCs involved in autoimmune diseases. Diagnostic and prognostic assays of the invention include methods for assessing the binding level of LtxA with WBCs.

The binding level in a test sample can be evaluated by obtaining a test sample from a test subject and contacting the test sample with LtxA. The "test sample" includes tissues, cells and biological fluids isolated from a subject, as well as tissues, cells and fluids present within a subject. The level of binding of LtxA to WBCs can be measured in a number of ways, including that described in the examples below. In a preferred embodiment, LtxA or its fragments that mediate binding between LtxA and LFA-1 (i.e., probes) are labeled with a detectable agent. The term "labeled" is intended to encompass direct labeling of the probe by physically linking a detectable substance to the probe, as well as indirect labeling of the probe by reactivity with a detectable substance. For example, LtxA (or its fragment) can be indirectly labeled using a second antibody directed against LtxA, wherein the second antibody is coupled to a detectable substance. Examples of detectable substances or labels include radio isotopes (e.g., ¹²⁵I, ¹³¹I, ³⁵S, ³H, or ³²P), enzymes (e.g., alkaline phosphatase, horseradish peroxidase, luciferase, or β-glactosidase), fluorescent moieties or proteins (e.g., fluorescein isothiocyanate, rhodamine, phycoerythrin, GFP, or BFP), or luminescent moieties (e.g., Qdot™ nanoparticles by the Quantum Dot Corporation, Palo Alto, CA).

LtxA not only binds to, but also kills, WBCs. In the diagnostic or prognostic method, to minimize any potential errors caused by cell death, the binding of LtxA and WBCs can be conducted at low temperatures (e.g., 0-4°C) and for a short period of time such as 5 to 20 or 30 minutes.

The prognostic assays described herein can be used to determine whether a subject can be administered with a cytotoxic drug or immune-suppressants to treat an autoimmune disorder, including those involved in organ/tissue transplantation.

Thus, also featured in this invention is a method of monitoring a treatment for autoimmune disorder in a subject. For this purpose, the binding level between LtxA and WBCs can be determined for test samples from a subject before, during, or after undergoing a treatment. A decrease in the binding level after the treatment indicates that the subject can be further treated by the same treatment. For example, a patient who has received organ or tissue transplantation often faces the problems of organ or tissue rejection. That is, the body has an immune response to an organ or tissue which causes failure of the transplant. To address this problem, organ or tissue transplantation is often accompanied by nonspecific immune suppression therapy to prevent T cell-mediated rejection. However, these immunosuppressants can cause infection, hypertension, cancer, and other undesirable side effects. Therefore, there is a need for monitoring the suppression. To that end, LtxA's biding level can serve as a marker for a proper level or degree of immune suppression. A skilled in the art can adjust the amount of immunosuppressants and length of treatment based on the level of the binding during the course of the treatment.

Information obtained from practice of the above assays is useful in prognostication, identifying progression of, and clinical management of diseases and other deleterious conditions affecting an individual's health status. The information more specifically assists the clinician in designing therapy or other treatment regimes to treat autoimmune diseases.

### EXAMPLE 1

In this example, LtxA's targeting of activated LFA-1 on WBCs was demonstrated in a study using a T-lymphocyte cell line (Jurkat) that expresses a high level of constitutively activated LFA-1 and control isogenic cells that either express the wild type form of the receptor or lack LFA-1 expression entirely. It was found that the T-lymphocytes were ten times more sensitive to LtxA-mediated toxicity than the control cells which were not affected.

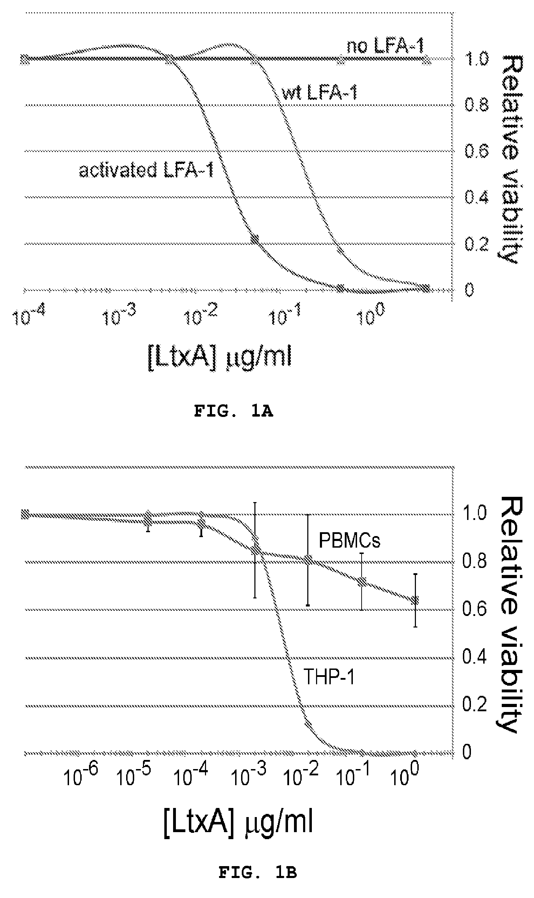

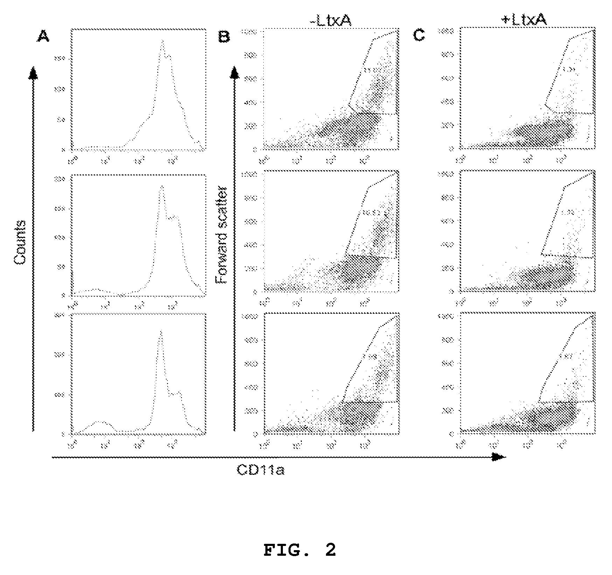

Malignant human monocyte cell line, THP-1, and peripheral blood mononuclear cells (PBMCs) PBMCs from four healthy adults were also treated with LtxA at different concentrations for 24 hours. Cell viability was then determined by measurement of cellular ATP. The results are shown in FIG. 1B. Untreated samples represent a relative viability of 1.0. The curve for PBMCs represents the average of the four human PBMC samples performed in quadruplicate. The vertical bars represent standard deviation. Results shown are representative of biological duplicates. As shown in FIG. 1B, LtxA had little effect on normal, resting human WBCs by examining its effect on normal PBMCs from healthy donors. The majority of cells were found to be resistant to LtxA and a drop in viability was only observed at very high drug concentrations. PBMCs prepared from healthy adults were also stained with anti-LFA-1 (CD11a) antibodies and analyzed by flow cytometry. Shown in FIG. 2 are results of cell size (forward scatter) vs. CD11a expression after 24-hour treatment with a control ("-LtxA") and LtxA ("LtxA"). As shown in the figures, only the activated cells with high levels of LFA-1 were affected by LtxA.

To determine whether LtxA can detect cells that had activated LFA-1 on their surface, purified LtxA was covalently labeled with a fluorescent substituent, fluorescein isothiocyanate (FITC). This allowed the LtxA to be detected and quantified by fluorescence spectroscopy when used in cell binding experiments. Laboratory studies showed that the LtxA-FITC retained full targeting and biological activity indicating that the FITC modification did not adversely affect toxin structure. Test cells were mixed with LtxA-FITC for 30 minutes at 0°C (on ice) and the resulting conjugates were analyzed by flow cytometry.

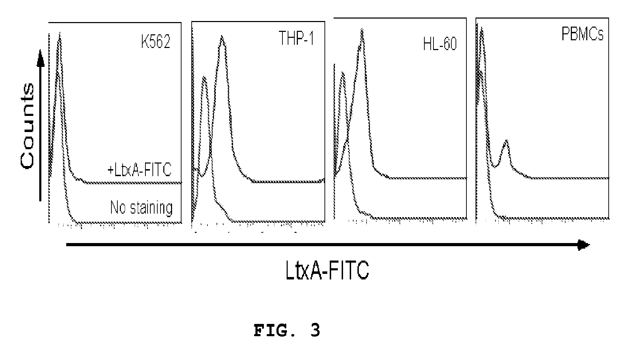

The test cells included K562 cells (which do not express LFA-1 and are unaffected by LtxA), THP-1 cells, HL-60 cells, and PBMCs (from a healthy donor). As shown in FIG. 3, LtxA-FITC did not kill the test cells due to the low temperatures and brief time period used. Further observations determined that K562 cells (which lack CD11a and CD18) did not bind the leukotoxin-FITC, indicating that LFA-1 is required for cell staining. In contrast, LtxA-FITC strongly attached itself to THP-1 cells and with slightly less intensity to HL-60 cells. Similar experiments were carried out with PBMCs, which possess minimal levels of activated surface LFA-1. As expected, it was found that only a small subset of the PBMCs were stained. These results demonstrate that LtxA binds to specific WBCs and that LtxA targets only cells with activated surface LFA-1.

### EXAMPLE 2

It was known that the initial steps leading to inflammation include the binding of activated PBMCs to endothelial cells and migration across the endothelial barrier. Strategies to treat inflammatory disorders therefore include depletion of those WBCs that are involved in pathogenesis and interruption of their binding and migration into the affected tissue. In this example, assays were carried out to determine if LtxA could block binding of activated PBMCs to human brain endothelial cells (HBECs) to model the initial steps leading to inflammation.

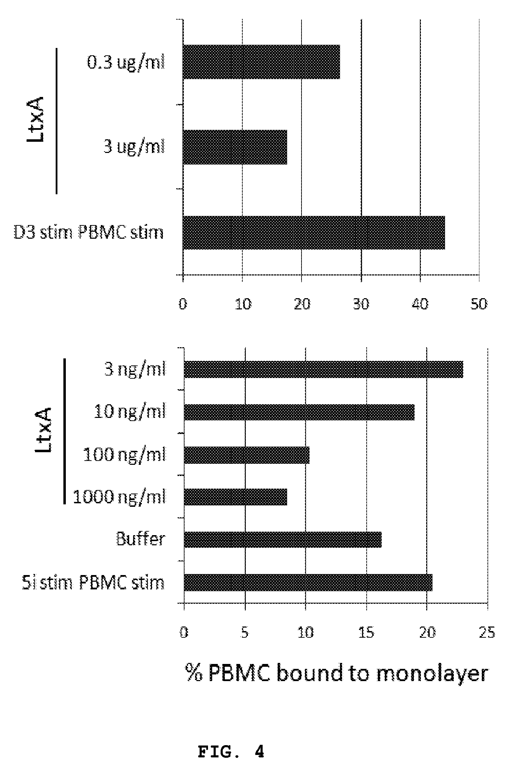

More specifically, D3 or 5i HBECs were grown to monolayer on collagen or gelatin and then stimulated with TNF. Calcein-labeled PBMCs activated with PMA were then added to the monolayer and incubated for 2 hours. Unbound cells were washed and the percent PBMC binding was calculated by measuring fluorescence. It was found that LtxA was able to prevent binding of activated PBMCs to the endothelial cells in a dose-dependent manner (FIG. 4). Greater than 50% blocking was observed at higher doses of LtxA. Because these PBMCs were from a healthy individual, complete blocking was not observed since most of the cells were normal and not affected by LtxA. Cytotoxicity assays showed that only approximately 10-20% of the cells stained with propidium iodide after two hours thereby indicating that many of the cells that were blocked by LtxA were not killed. Thus, the above results demonstrate that LtxA has the ability to selectively deplete and block activated PBMCs from binding to endothelial cells.

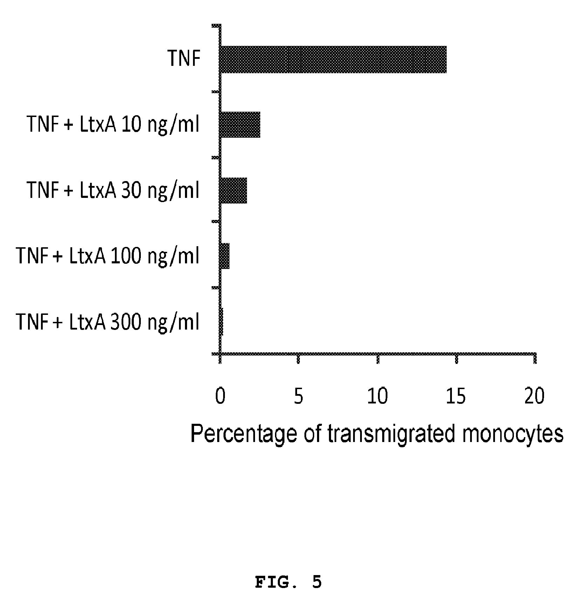

In addition, in an *in vitro* model for multiple sclerosis (MS), it was found that LtxA blocked the migration of monocytes across a HBEC layer (FIG. 5). This model represents migration of WBCs into the CNS, which occurs in MS. These results suggest that LtxA can be used in treating MS via its ability to selectively deplete and/or block monocytes from entering into the CNS.

### EXAMPLE 3

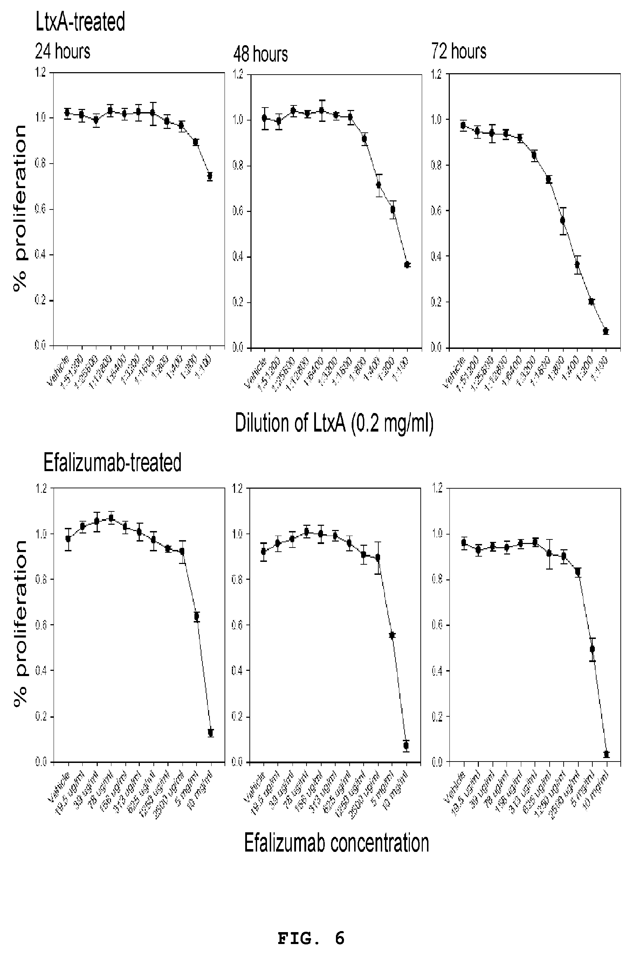

In this example, assays were carried out to compare the effects of LtxA and anti-LFA-1 mAb Efalizumab on T-cells. More specifically, 5x10⁵ Jurkat cells/ml were treated with serial dilutions of LtxA or Efalizumab for 72 hours before the proliferation of the cells were examined using the CellTiter Glo kit from PROMEGA.. It was found that LtxA decreased proliferation of the cells in a dose-dependent manner (FIG. 6). The inhibition of proliferation increased over time with an IC₅₀ value of 250 ng/ml after 72 hours. Efalizumab also decreased proliferation of the cells in a dose-dependent manner; but the time of stimulation did not seem to augment this effect (FIG. 6). The IC₅₀ value for Efalizumab was 5 mg/ml at all times. Thus, the effective dose of LtxA is approximately 20,000 times lower than that of Efalizumab.

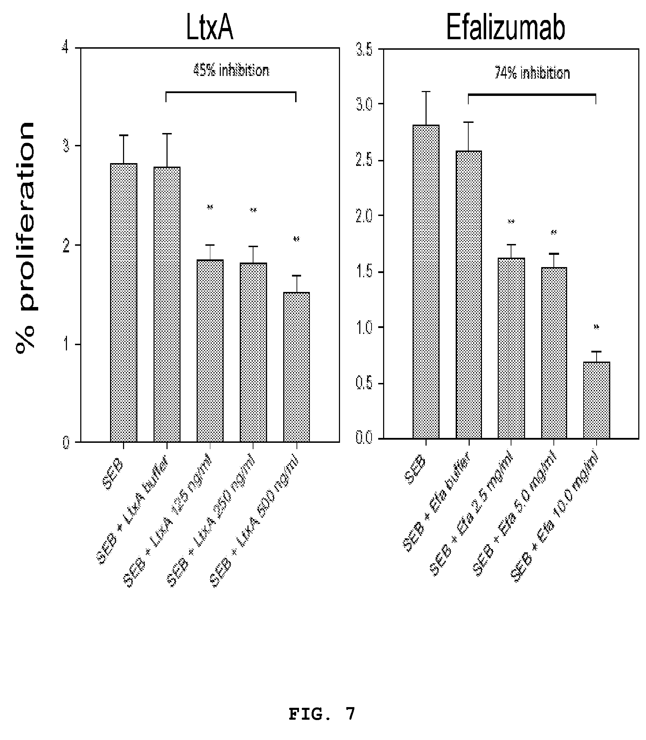

Assays were also carried out to examine the efficacy of LtxA and Efalizumab on activated PBMCs from psoriasis patients. Briefly, PBMCs were isolated from ten donors with severe plaque psoriasis (three females and seven males, age 29-63 (48±13)). PBMCs were isolated from blood samples by centrifugation over a lymphoprep density gradient and immediately frozen in liquid nitrogen until used. The cells were then activated using Staphylococcal enterotoxin B (SEB) at a final concentration of 1 µg/ml and treated with dilutions of LtxA or Efalizumab. It was found that LtxA inhibited proliferation of the activated PBMCs from psoriasis patients and the effective doses were at least 5000 times lower than those of Efalizumab (FIG. 7).

### EXAMPLE 4

In this example, assays were carried out to evaluate the effect of LtxA and Efalizumab in alleviating psoriasis *in vivo* in a psoriasis xenograft transplantation model. As mentioned above, LFA-1 is a heterodimer consisting of CD11a (αL) and CD18 (β2) integrin subunits. It binds to intercellular adhesion molecule 1 (ICAM-1) present on antigen-presenting cells (APCs) and functions as an adhesion molecule. Binding between LFA-1 and ICAM-1 results in induction of T-cell activation, but also allows anchoring of these cells to the endothelium which is followed by extravasation leading to recruitment of T-cells to the site of inflammation.

### Methods

### Patient material:

Two patients with plaques-type psoriasis were identified and keratome skin biopsies from lesional skin were obtained. The patient's psoriasis was un-treated for at least 1 month prior to the time of skin removal.

### Xenograft transplantation protocol:

Keratome skin biopsies were cut into smaller pieces before transplantation on the back of anesthetized SCID mice (female, 6-8 week of age, M&B Taconic, Denmark). After a healing period of approximately 10 days, the animals were divided into separate treatment groups. A total of 24 mice were entered into the study. Animals were allocated to two consecutive study series, each representing skin grafts from one individual psoriasis patient (total of 2 patients, 12 mice for each series). Each series was subdivided into 4 groups. One group in each series served as untreated control whereas the other groups were allocated to treatment with LtxA, efalizumab, or LtxA vehicle. The allocation scheme was as follows:

| Group | Mice pr group per series | Treatment | Total mice per treatment |
|---|---|---|---|
| 1 | 2 | untreated | 4 |
| 2 | 4 | Efalizumab | 8 |
| 3 | 4 | LtxA | 8 |
| 4 | 2 | LtxA vehicle | 4 |

LtxA (0.5 mg/kg), efalizumab (6 mg/kg), and LtxA vehicle (Tris buffer/NaCl) was administered once daily by i.p. injection for 3 weeks. These dosages were chosen as it was demonstrated that 2 mg/kg LtxA had a non-toxic therapeutic effect when it was administered in a mouse leukemia xenograft model (Kachlany, S. C. et al.,. Leukemia Research 2010; 34:777-85.). Similarly, administration of 6 mg/kg efalizumab (i.p. daily) or 10 mg/kg anti-ICAM-1 (i.p. every second day) to mice in the psoriasis xenograft transplantation model demonstrated a therapeutic effect (Zeigler M et. al., Lab Invest 2001; 81: 1253-61 and Boehncke WH et. al., Br J Dermatol 2005; 153: 758-66).

During treatment, human psoriatic skin grafts were clinically assessed twice weekly and given a semi-quantitative clinical psoriasis score according to the clinical signs: scaliness, induration, and erythema. The parameters were scored using the three-point scale: 0 = complete lack of cutaneous involvement; 1 = slight involvement; 2 = moderate involvement; 3 = severe involvement. On this scale from 0 to 3, a maximal score of 3 represents severe scale, induration, and erythema of the psoriatic xenografts.

After 3 weeks and after the final clinical assessment, the animals were killed and 4-mm size punch biopsies were taken centrally from each human psoriatic skin graft. The biopsy samples were paraffin embedded and stained with haematoxylin/eosin (HE), On five HE stained sections the following parameters were assessed: 1) epidermal thickness, 2) parakeratosis, 3) psoriasis pattern, 4) angiogenesis, 5) lymphocytes, and 6) stratum granulosum. Epidermal thickness was measured as the distance from stratum corneum to the deepest part of the rete pegs. Parakeratosis, psoriasis pattern, angiogenesis and lymphocytes were evaluated and given scores in the range 0-4 where 0 denotes no psoriasis and 4 denotes full fledge psoriasis. Stratum granulosum was evaluated in the range 0-4 where 0 denotes full fledge psoriasis and 4 no psoriasis.

### Statistic analysis:

Results are shown as mean ± SEM. The non-parametric Mann Whitney test was used to test for differences between treatment groups in semiquantitative clinical psoriasis scores, parakeratosis scores, psoriasis pattern scores, angiogenesis scores, lymphocyte scores, and stratum granulosum scores. Students t-test was used to test for no differences between treatment groups for epidermal thickness. Observations made for different mice were assumed to be independent of each other. All tests were two-sided and p values < 0.05 were considered significant.

### Results

### Control Treatment (untreated versus LtxA vehicle):

No differences between the vehicle and the untreated groups were found in the semi-quantitative clinical psoriasis, the parakeratosis, the psoriasis pattern, the angiogenesis, the lymphocyte, the stratum granulosum scores, and the epidermal thickness measures for both patients. Therefore these two groups are pooled in the following and named negative control group.

Also, it was found that the mice treated with LtxA did not show any physiological changes during the study period, and their bodyweight increased throughout the study similarly to the negative control treated mice.

### Semiquantitative clinical psoriasis score:

Semi-quantitative clinical psoriasis scores provide a superficial evaluation of the graft where the histological scores show a more in depth status. Reduction in epidermal thickness is considered the final endpoint when evaluating the treatment effect.

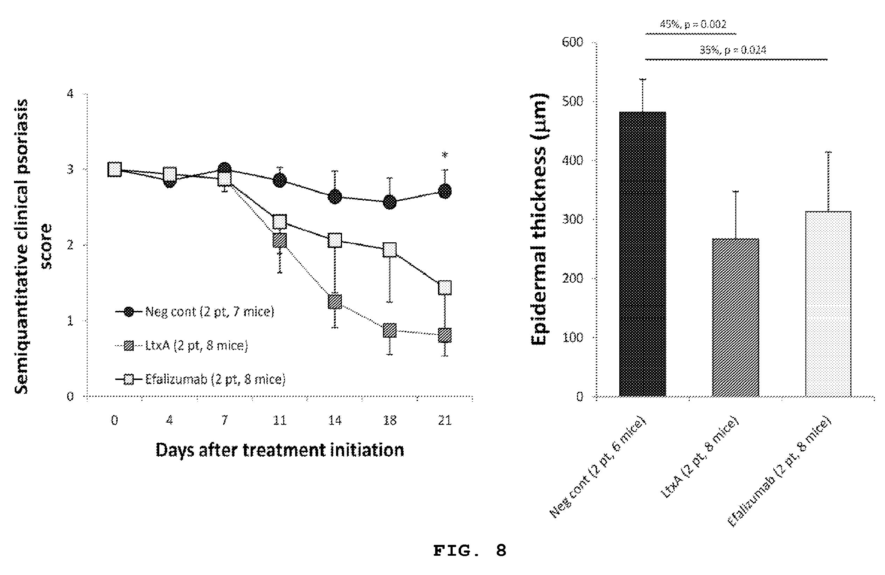

Semiquantitative clinical psoriasis scores were obtained twice weekly throughout the study. As shown in FIG. 8, LtxA significantly decreased the semiquantitative clinical psoriasis score (p < 0.001) by 3 weeks treatment. Efalizumab also decreased the semiquantitative clinical psoriasis score (p = 0.094) by 3 weeks treatment, however not to a significant degree.

### Epidermal thickness:

After 3 weeks treatment, the mice were killed and biopsies taken from the human psoriatic skin graft. Epidermal thickness was measured on HE stained paraffin embedded sections. It was found that LtxA (p = 0.002) and efalizumab (p = 0.024) significantly decreased the epidermal thickness. See FIG. 8.

### Psoriasis pattern scores:

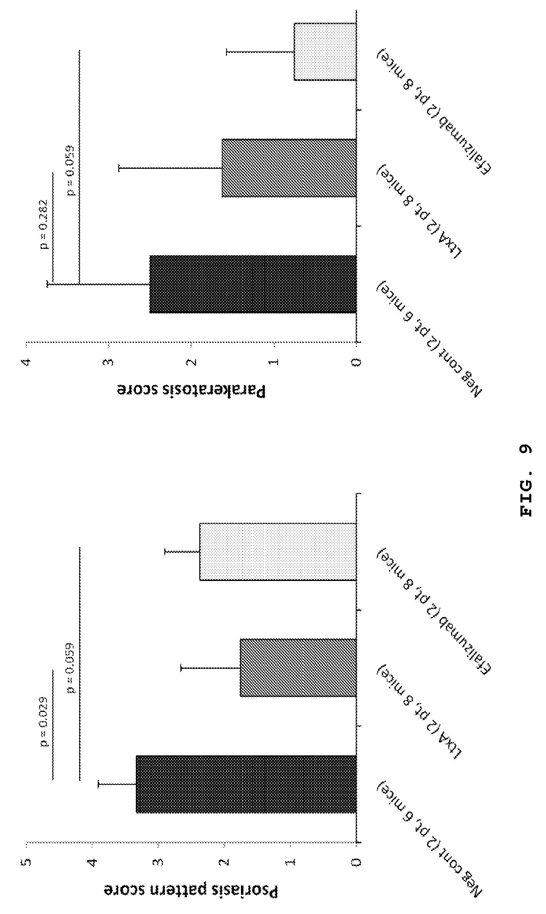

Psoriasis pattern scores give an overall assessment of the psoriatic phenotype observed in the HE stained sections and sums up the results of the epidermal thickness measure, the parakeratosis, the angiogenesis, the lymphocyte, and the stratum granulosum scores. As shown in FIG. 9, LtxA significantly decreased the psoriasis pattern score (p = 0.029) by 3 weeks treatment. Efalizumab also decreased the psoriasis pattern score (p = 0.059) by 3 weeks treatment, however not to a significant degree.

### Parakeratosis scores:

The parakeratosis scores provide an assessment of the degree of parakeratosis observed in epidermis. Due to the increased turnover and decreased differentiation of keratinocytes in psoriatic skin, parakeratosis (presence of nucleus in desquamated cells) is often present in psoriatic skin. In this study, it was found that LtxA (p = 0.282) and efalizumab (p = 0.059) decreased the parakeratosis score by 3 weeks treatment, however not to a significant degree. See FIG. 9.

### Angiogenesis scores:

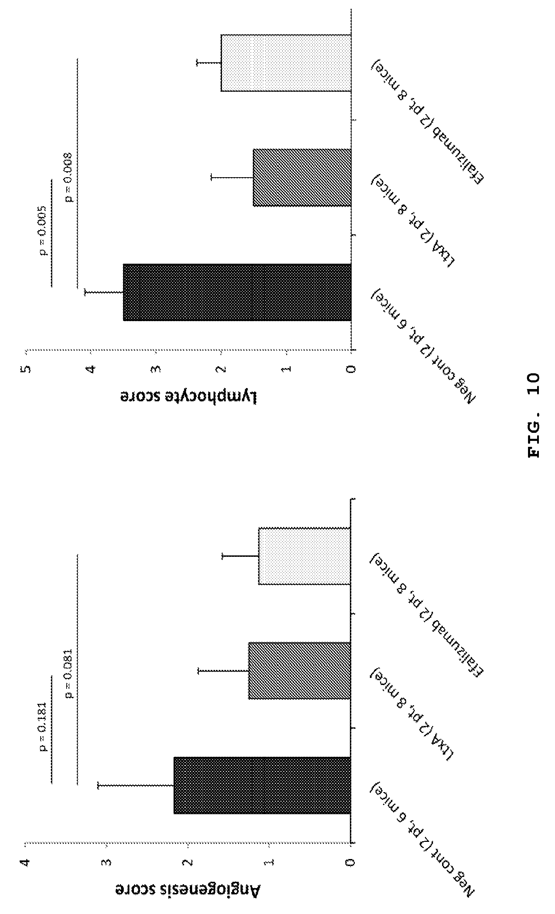

Angiogenesis scores provide an assessment of the degree of vascularization observed in the dermal compartment. Dermis of psoriatic skin is highly vascularized as compared to the dermis of healthy skin. In this study, it was found that LtxA (p = 0.181) and efalizumab (p = 0.081) decreased the angiogenesis score by 3 weeks treatment, however not to a significant degree See FIG. 10.

### Lymphocyte scores:

Lymphocyte scores provide an assessment of the degree of lymphocytic infiltrate present both in the dermal and the epidermal compartment. Psoriatic skin is characterized by an increased infiltrate of lymphocytes compared to healthy skin. In this study, it was found that LtxA (p = 0.005) and efalizumab (p = 0.008) both significantly decreased the lymphocyte score by 3 weeks treatment. See FIG. 10.

### Stratum granulosum scores:

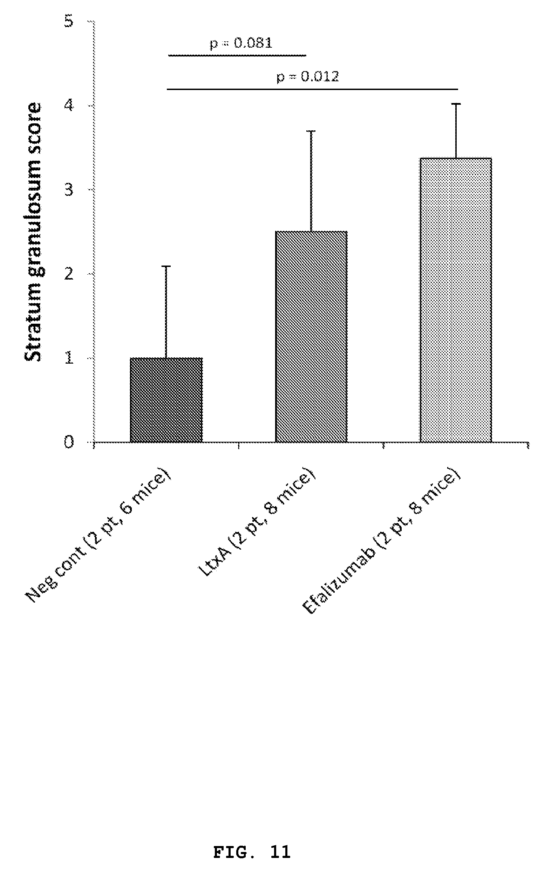

Stratum granulosum scores provide an assessment of the degree of stratum granulosum presence in the epidermis. Due to the increased turnover and decreased differentiation of keratinocytes in psoriatic skin, this cell layer is typically diminished or lost in psoriatic skin. In this study, it was found that LtxA decreased the stratum granulosum score (p = 0.081) by 3 weeks treatment, however not to a significant degree. Efalizumab significantly decreased the stratum granulosum score (p = 0.012) by 3 weeks treatment. See FIG. 11.

In sum, the above results demonstrated that the three-week treatment with LtxA significantly decreased the semi-quantitative clinical psoriasis score, the epidermal thickness, psoriasis pattern, and the lymphocyte scores. Efalizumab significantly decreased the epidermal thickness and the lymphocyte scores. Also, this study demonstrated that LtxA significantly alleviated the psoriatic phenotype in the grafted skin from both patients. Efalizumab significantly alleviated the epidermal thickness but not the clinical phenotype of psoriasis. Thus, the results suggest that LtxA is more effective than Efalizumab in treating psoriasis.

## Claims

1. Leukotoxin LtxA (SEQ ID NO:1) or a polypeptide having at least 95% sequence identity with SEQ ID NO:1 and retaining the activity of the LtxA polypeptide for use in treating a disorder selected from the group consisting of psoriasis, Crohn's disease, ulcerative colitis, multiple sclerosis, rheumatoid arthritis, systemic lupus erythematosus (SLE) and transplant rejection.

2. Leukotoxin LtxA or polypeptide for use according to claim 1, wherein said leukotoxin or said polypeptide is prepared from *Aggregatibacter actinomycetemcomitans.*

3. Leukotoxin LtxA or polypeptide for use according to claim 1, wherein the disorder is psoriasis.

4. A pharmaceutical composition comprising as the sole active ingredient leukotoxin LtxA (SEQ ID NO:1) or a polypeptide having at least 95% identity to SEQ ID NO:1 and retaining the activity of the LtxA polypeptide, said pharmaceutical composition further comprising a pharmaceutical carrier, for use in treating a disorder selected from the group consisting of psoriasis, Crohn's disease, ulcerative colitis, multiple sclerosis, rheumatoid arthritis, systemic lupus erythematosus (SLE) and transplant rejection.

5. The pharmaceutical composition for use according to claim 4, wherein said leukotoxin or said polypeptide is prepared from *Aggregatibacter actinomycetemcomitans.*

6. A method for determining whether a subject has a disorder selected from the group consisting of psoriasis, Crohn's disease, ulcerative colitis, multiple sclerosis, rheumatoid arthritis, systemic lupus erythematosus (SLE) and transplant rejection, said method comprising:
contacting a sample obtained from a subject, said sample containing white blood cells, with leukotoxin LtxA (SEQ ID NO:1) or with a polypeptide having at least 95% identity to SEQ ID NO:1 and retaining the activity of the LtxA polypeptide; and
determining the percentage of the white blood cells that bind to said leukotoxin or to said polypeptide in the sample;
wherein the subject is determined to have the disorder if the percentage is at or above a predetermined value.

7. The method of claim 6, wherein said leukotoxin or said polypeptide is labeled with a detectable agent.

8. The method of claim 7, wherein the detectable agent is FITC.

9. The method of claim 6,
wherein the sample is a blood sample, or
wherein the contacting step is conducted at about 0-4 degrees centigrade, or
wherein the predetermined value is obtained from a control subject that does not have the disorder.

10. The method of claim 6, wherein the disorder is psoriasis.

11. A method for determining the effectiveness of a treatment in a patient suffering from a disorder selected from the group consisting of psoriasis, Crohn's disease, ulcerative colitis, multiple sclerosis, rheumatoid arthritis, systemic lupus erythematosus (SLE) and transplant rejection, the method comprising:
contacting a sample obtained from a patient that has received a treatment, said sample containing white blood cells, with leukotoxin LtxA (SEQ ID NO:1) or with a polypeptide having at least 95% identity to SEQ ID NO:1 and retaining the activity of the LtxA polypeptide; and
determining the percentage of the white blood cells that bind to said leukotoxin or to said polypeptide in the sample;
wherein the treatment is determined to be effective if the percentage is below a predetermined value.

12. The method of claim 11, wherein said leukotoxin or said polypeptide is labeled with a detectable agent.

13. The method of claim 12, wherein the detectable agent is FITC.

14. The method of claim 11,
wherein the sample is a blood sample, or
wherein the contacting step is conducted at about 0-4 degrees centigrade, or
wherein the predetermined value is a control value obtained from the patient prior to the treatment.

15. The method of claim 11, wherein the disorder is psoriasis.

## Patentansprüche

1. Leukotoxin LtxA (SEQ ID NR. 1) oder ein Polypeptid, das mindestens 95 % Sequenzidentität mit SEQ ID NR. 1 aufweist und die Aktivität des LtxA-Polypeptids beibehält, zur Verwendung beim Behandeln einer Erkrankung, die aus der Gruppe ausgewählt ist, welche aus Psoriasis, Morbus Crohn, Colitis ulcerosa, multipler Sklerose, rheumatoider Arthritis, systemischem Lupus erythematodes (SLE) und Transplantatabstoßung besteht.

2. Leukotoxin LtxA oder Polypeptid zur Verwendung nach Anspruch 1, wobei das Leukotoxin oder das Polypeptid von *Aggregatibacter actinomycetemcomitans* gewonnen wird.

3. Leukotoxin LtxA oder Polypeptid zur Verwendung nach Anspruch 1, wobei es sich bei der Erkrankung um Psoriasis handelt.

4. Pharmazeutische Zusammensetzung, umfassend als alleinigen Wirkstoff Leukotoxin LtxA (SEQ ID NR. 1) oder ein Polypeptid, das mindestens 95 % Sequenzidentität mit SEQ ID NR. 1 aufweist und die Aktivität des LtxA-Polypeptids beibehält, wobei die pharmazeutische Zusammensetzung ferner einen pharmazeutischen Träger aufweist, zur Verwendung beim Behandeln einer Erkrankung, die aus der Gruppe ausgewählt ist, welche aus Psoriasis, Morbus Crohn, Colitis ulcerosa, multipler Sklerose, rheumatoider Arthritis, systemischem Lupus erythematodes (SLE) und Transplantatabstoßung besteht.

5. Pharmazeutische Zusammensetzung zur Verwendung nach Anspruch 4, wobei das Leukotoxin oder das Polypeptid von *Aggregatibacter actinomycetemcomitans* gewonnen wird.

6. Verfahren zum Bestimmen, ob ein Individuum eine Erkrankung hat, die aus der Gruppe ausgewählt ist, welche aus Psoriasis, Morbus Crohn, Colitis ulcerosa, multipler Sklerose, rheumatoider Arthritis, systemischem Lupus erythematodes (SLE) und Transplantatabstoßung besteht, wobei das Verfahren Folgendes umfasst:
Inkontaktbringen einer von einem Individuum erhaltenen Probe, wobei die Probe weiße Blutzellen enthält, mit Leukotoxin LtxA (SEQ ID NR. 1) oder mit einem Polypeptid, das mindestens 95 % Sequenzidentität mit SEQ ID NR. 1 aufweist und die Aktivität des LtxA-Polypeptids beibehält; und
Bestimmen des Prozentanteils der weißen Blutzellen, die an das Leukotoxin oder an das Polypeptid binden, in der Probe;
wobei festgestellt wird, dass das Individuum die Erkrankung hat, wenn der Prozentanteil einem im Voraus festgelegten Wert entspricht oder darüber liegt.

7. Verfahren nach Anspruch 6, wobei das Leukotoxin oder das Polypeptid mit einem detektierbaren Mittel markiert ist.

8. Verfahren nach Anspruch 7, wobei es sich bei dem detektierbaren Mittel um FITC handelt.

9. Verfahren nach Anspruch 6,
wobei es sich bei der Probe um eine Blutprobe handelt oder
wobei der Schritt des Inkontaktbringens bei ungefähr 0 bis 4 Grad Celsius durchgeführt wird oder
wobei der im Voraus festgelegte Wert von einem Kontrollindividuum erhalten wird, das nicht an der Erkrankung leidet.

10. Verfahren nach Anspruch 6, wobei es sich bei der Erkrankung um Psoriasis handelt.

11. Verfahren zum Bestimmen der Wirksamkeit einer Behandlung bei einem Patienten/einer Patientin, der/die an einer Erkrankung leidet, die aus der Gruppe ausgewählt ist, welche aus Psoriasis, Morbus Crohn, Colitis ulcerosa, multipler Sklerose, rheumatoider Arthritis, systemischem Lupus erythematodes (SLE) und Transplantatabstoßung besteht, wobei das Verfahren Folgendes umfasst:
Inkontaktbringen einer Probe von einem Patienten/einer Patientin, der/die eine Behandlung erhalten hat, wobei die Probe weiße Blutzellen enthält, mit Leukotoxin LtxA (SEQ ID NR. 1) oder mit einem Polypeptid, das mindestens 95 % Sequenzidentität mit SEQ ID NR. 1 aufweist und die Aktivität des LtxA-Polypeptids beibehält; und
Bestimmen des Prozentanteils der weißen Blutzellen, die an das Leukotoxin oder an das Polypeptid binden, in der Probe;
wobei festgestellt wird, dass die Behandlung wirksam ist, wenn der Prozentanteil unter einem im Voraus festgelegten Wert liegt.

12. Verfahren nach Anspruch 11, wobei das Leukotoxin oder das Polypeptid mit einem detektierbaren Mittel markiert ist.

13. Verfahren nach Anspruch 12, wobei es sich bei dem detektierbaren Mittel um FITC handelt.

14. Verfahren nach Anspruch 11,
wobei es sich bei der Probe um eine Blutprobe handelt oder
wobei der Schritt des Inkontaktbringens bei ungefähr 0 bis 4 Grad Celsius durchgeführt wird oder
wobei es sich bei dem im Voraus festgelegten Wert um einen Kontrollwert handelt, der vor der Behandlung von dem Patienten erhalten wird.

15. Verfahren nach Anspruch 11, wobei es sich bei der Erkrankung um Psoriasis handelt.

## Revendications

1. Leukotoxine LtxA (SEQ ID NO : 1) ou un polypeptide possédant au moins 95% d'identité de séquence avec SEQ ID NO : 1 et conservant l'activité du polypeptide LtxA pour une utilisation dans le traitement d'un trouble choisi dans le groupe constitué du psoriasis, de la maladie de Crohn, de la rectocolite hémorragique, de la sclérose en plaques, de l'arthrite rhumatoïde, du lupus érythémateux systémique (LES) et du rejet de greffe.

2. Leukotoxine LtxA ou polypeptide selon la revendication 1, dans lequel ladite leukotoxine ou ledit polypeptide est préparé à partir d*'Aggregatibacter actinomycetemcomitans.*

3. Leukotoxine LtxA ou polypeptide pour une utilisation selon la revendication 1, dans lequel le trouble est le psoriasis.

4. Composition pharmaceutique comprenant en tant qu'unique ingrédient actif la leukotoxine LtxA (SEQ ID NO : 1) ou un polypeptide possédant au moins 95% d'identité de séquence avec SEQ ID NO : 1 et conservant l'activité du polypeptide LtxA, ladite composition pharmaceutique comprenant en outre un véhicule pharmaceutique, pour une utilisation dans le traitement d'un trouble choisi dans le groupe constitué du psoriasis, de la maladie de Crohn, de la rectocolite hémorragique, de la sclérose en plaques, de l'arthrite rhumatoïde, du lupus érythémateux systémique (LES) et du rejet de greffe.

5. Composition pharmaceutique pour une utilisation selon la revendication 4, dans laquelle ladite leukotoxine ou ledit polypeptide est préparé à partir d*'Aggregatibacter actinomycetemcomitans.*

6. Procédé pour déterminer si un sujet a un trouble choisi dans le groupe constitué du psoriasis, de la maladie de Crohn, de la rectocolite hémorragique, de la sclérose en plaques, de l'arthrite rhumatoïde, du lupus érythémateux systémique (LES) et du rejet de greffe, ledit procédé comprenant les étapes consistant à :
mettre en contact un échantillon obtenu à partir d'un sujet, ledit échantillon contenant des globules blancs, avec la leukotoxine LtxA (SEQ ID NO : 1) ou un polypeptide possédant au moins 95% d'identité de séquence avec SEQ ID NO : 1 et conservant l'activité du polypeptide LtxA ; et
déterminer le pourcentage de globules blancs qui se lient à ladite leukotoxine ou audit polypeptide dans l'échantillon ;
dans lequel le sujet est considéré comme ayant le trouble si le pourcentage est égal ou supérieur à une valeur prédéterminée.

7. Procédé selon la revendication 6, dans lequel ladite leukotoxine ou ledit polypeptide est marqué avec un agent détectable.

8. Procédé selon la revendication 7, dans lequel l'agent détectable est FITC.

9. Procédé selon la revendication 6,
dans lequel l'échantillon est un échantillon de sang, ou
dans lequel l'étape de mise en contact est effectuée d'environ 0 à 4 degrés centigrade, ou
dans lequel la valeur prédéterminée est obtenue à partir d'un sujet de contrôle qui ne présente pas le trouble.

10. Procédé selon la revendication 6, dans lequel le trouble est le psoriasis.

11. Procédé pour déterminer l'efficacité d'un traitement chez un patient souffrant d'un trouble choisi dans le groupe constitué du psoriasis, de la maladie de Crohn, de la rectocolite hémorragique, de la sclérose en plaques, de l'arthrite rhumatoïde, du lupus érythémateux systémique (LES) et du rejet de greffe, ledit procédé comprenant les étapes consistant à :
mettre en contact un échantillon obtenu à partir d'un sujet qui a reçu un traitement, ledit échantillon contenant des globules blancs, avec la leukotoxine LtxA (SEQ ID NO : 1) ou avec un polypeptide possédant au moins 95% d'identité de séquence avec SEQ ID NO : 1 et conservant l'activité du polypeptide LtxA ; et
déterminer le pourcentage de globules blancs qui se lient à ladite leukotoxine ou audit polypeptide dans l'échantillon ;
dans lequel le traitement est considéré comme étant efficace si le pourcentage est inférieur à une valeur prédéterminée.

12. Procédé selon la revendication 11, dans lequel ladite leukotoxine ou ledit polypeptide est marqué avec un agent détectable.

13. Procédé selon la revendication 12, dans lequel l'agent détectable est FITC.

14. Procédé selon la revendication 11,
dans lequel l'échantillon est un échantillon de sang, ou
dans lequel l'étape de mise en contact est effectuée d'environ 0 à 4 degrés centigrade, ou
dans lequel la valeur prédéterminée est une valeur de contrôle obtenue à partir du patient avant le traitement.

15. Procédé selon la revendication 11, dans lequel le trouble est le psoriasis.
